(19) **Europäisches Patentamt European Patent Office Office européen des brevets**

(11) **EP 3 827 428 B1**

(12) # EUROPEAN PATENT SPECIFICATION

(45) Date of publication and mention of the grant of the patent:
**30.10.2024 Bulletin 2024/44**

(21) Application number: **19839971.9**

(22) Date of filing: **24.07.2019**

(51) International Patent Classification (IPC):
**G10L 21/00** (2013.01)  **H04R 3/00** (2006.01)
**H04R 25/00** (2006.01)  **A61N 1/36** (2006.01)

(52) Cooperative Patent Classification (CPC):
**A61N 1/36038; G10L 21/00; G10L 25/51; H04R 25/507;** G10L 25/18; G10L 25/30

(86) International application number:
**PCT/US2019/043160**

(87) International publication number:
**WO 2020/023585 (30.01.2020 Gazette 2020/05)**

(54) **NEURAL NETWORK AUDIO SCENE CLASSIFIER FOR HEARING IMPLANTS**

KLASSIERER VON AUDIOSZENEN EINES NEURONALEN NETZES FÜR HÖRIMPLANTATE

CLASSIFICATEUR DE SCÈNES AUDIO À RÉSEAU NEURONAL POUR IMPLANTS AUDITIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2018 US 201862703490 P**

(43) Date of publication of application:
**02.06.2021 Bulletin 2021/22**

(73) Proprietor: **MED-EL Elektromedizinische Geräte GmbH**
**6020 Innsbruck (AT)**

(72) Inventors:
• **MARTIN, Rainer**
**44801 Bochum (DE)**
• **AGCAER, Semih**
**44801 Bochum (DE)**
• **FRÜHAUF, Florian**
**6074 Rinn (AT)**
• **ASCHBACHER, Ernst**
**6020 Innsbruck (AT)**
• **RANK, Erhard**
**6020 Innsbruck (AT)**

(74) Representative: **Lucke, Andreas**
**Boehmert & Boehmert**
**Anwaltspartnerschaft mbB**
**Pettenkoferstrasse 22**
**80336 München (DE)**

(56) References cited:
WO-A1-2016/110804    WO-A1-2020/126028
CN-A- 108 231 067    US-A1- 2017 001 006
US-A1- 2017 178 666    US-A1- 2017 311 095
US-B1- 7 164 771

• **DELIANG WANG: "Deep Learning Reinvents the Hearing Aid -", IEEE SPECTRUM, 1 December 2016 (2016-12-01), pages 1 - 8, XP055682346, Retrieved from the Internet <URL:https://spectrum.ieee.org/consumer-electronics/audiovideo/deep-learning-reinvents-the-hearing-aid> [retrieved on 20200402], DOI: 10.1109/MSPEC.2017.7864754**
• **CROCCO MARCO MARCO CROCCO@IIT IT ET AL: "Audio Surveillance", ACM COMPUTING SURVEYS, ACM, NEW YORK, NY, US, US, vol. 48, no. 4, 22 February 2016 (2016-02-22), pages 1 - 46, XP058666229, ISSN: 0360-0300, DOI: 10.1145/2871183**
• **BARCHIESI DANIELE ET AL: "Acoustic Scene Classification: Classifying environments from the sounds they produce", IEEE SIGNAL PROCESSING MAGAZINE, IEEE, USA, vol. 32, no. 3, 1 May 2015 (2015-05-01), pages 16 - 34, XP011577488, ISSN: 1053-5888, [retrieved on 20150402], DOI: 10.1109/MSP.2014.2326181**

- **WANG, DELIANG: "Deep Learning Reinvents the Hearing Aid", SPECTRUM.IEEE.ORG I NORTH AMERICAN, December 2016 (2016-12-01), pages 32 - 37, XP055682346, Retrieved from the Internet <URL:http://www.nxtbook.com/nxtbooks/ieee/spectrum-na_0317/index.php#/34> [retrieved on 20190914]**
- **DLAMINI, GCINIWE: "Machine Learning Methods for Individual Acoustic Recognition in a Species of Field Cricket", DISSERTATION, February 2018 (2018-02-01), pages 1 - 100, XP055682350, Retrieved from the Internet <URL:https://open.uct.ac.za/bitstream/handle/11427/29619/thesis_sci_2018_diamini_gciniwe.pdf?sequence=1&isAllowed=y> [retrieved on 20190915]**

**Description**

TECHNICAL FIELD

The present invention is set out in the appended claims and generally relates to hearing implant systems such as cochlear implants, and specifically to the signal processing used therein associated with audio scene classification.

BACKGROUND ART

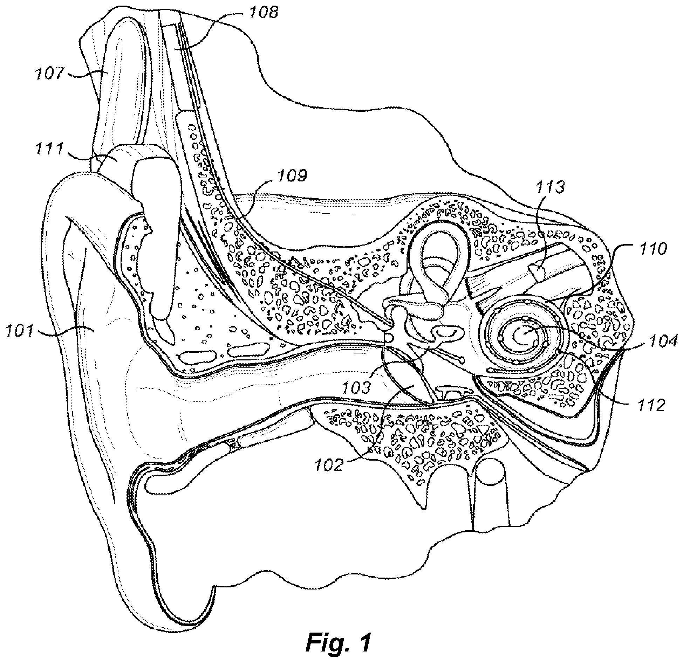

A normal ear transmits sounds as shown in Figure 1 through the outer ear **101** to the tympanic membrane **102,** which moves the bones of the middle ear **103** (malleus, incus, and stapes) that vibrate the oval window and round window openings of the cochlea **104.** The cochlea **104** is a long narrow duct wound spirally about its axis for approximately two and a half turns. It includes an upper channel known as the scala vestibuli and a lower channel known as the scala tympani, which are connected by the cochlear duct. The cochlea **104** forms an upright spiraling cone with a center called the modiolar where the spiral ganglion cells of the acoustic nerve **113** reside. In response to received sounds transmitted by the middle ear **103,** the fluid-filled cochlea **104** functions as a transducer to generate electric pulses which are transmitted to the cochlear nerve **113,** and ultimately to the brain.

Hearing is impaired when there are problems in the ability to transduce external sounds into meaningful action potentials along the neural substrate of the cochlea **104.** To improve impaired hearing, hearing prostheses have been developed. For example, when the impairment is related to operation of the middle ear **103,** a conventional hearing aid may be used to provide mechanical stimulation to the auditory system in the form of amplified sound. Or when the impairment is associated with the cochlea **104,** a cochlear implant with an implanted stimulation electrode can electrically stimulate auditory nerve tissue with small currents delivered by multiple electrode contacts distributed along the electrode.

Figure 1 also shows some components of a typical cochlear implant system, including an external microphone that provides an audio signal input to an external signal processor **111** where various signal processing schemes can be implemented. The processed signal is then converted into a digital data format, such as a sequence of data frames, for transmission into the implant **108.** Besides receiving the processed audio information, the implant **108** also performs additional signal processing such as error correction, pulse formation, etc., and produces a stimulation pattern (based on the extracted audio information) that is sent through an electrode lead **109** to an implanted electrode array **110.**

Typically, the electrode array **110** includes multiple electrode contacts **112** on its surface that provide selective stimulation of the cochlea **104.** Depending on context, the electrode contacts **112** are also referred to as electrode channels. In cochlear implants today, a relatively small number of electrode channels are each associated with relatively broad frequency bands, with each electrode contact **112** addressing a group of neurons with an electric stimulation pulse having a charge that is derived from the instantaneous amplitude of the signal envelope within that frequency band.

It is well-known in the field that electric stimulation at different locations within the cochlea produce different frequency percepts. The underlying mechanism in normal acoustic hearing is referred to as the tonotopic principle. In cochlear implant users, the tonotopic organization of the cochlea has been extensively investigated; for example, see Vermeire et al., Neural tonotopy in cochlear implants: An evaluation in unilateral cochlear implant patients with unilateral deafness and tinnitus, Hear Res, 245(1-2), 2008 Sep 12 p. 98-106; and Schatzer et al., Electric-acoustic pitch comparisons in single-sided-deaf cochlear implant users: Frequency-place functions and rate pitch, Hear Res, 309, 2014 Mar, p. 26-35.

In some stimulation signal coding strategies, stimulation pulses are applied at a constant rate across all electrode channels, whereas in other coding strategies, stimulation pulses are applied at a channel-specific rate. Various specific signal processing schemes can be implemented to produce the electrical stimulation signals. Signal processing approaches that are well-known in the field of cochlear implants include continuous interleaved sampling (CIS), channel specific sampling sequences (CSSS) (as described in U.S. Patent No. 6,348,070), spectral peak (SPEAK), and compressed analog (CA) processing.

In the CIS strategy, the signal processor only uses the band pass signal envelopes for further processing, i.e., they contain the entire stimulation information. For each electrode channel, the signal envelope is represented as a sequence of biphasic pulses at a constant repetition rate. A characteristic feature of CIS is that the stimulation rate is equal for all electrode channels and there is no relation to the center frequencies of the individual channels. It is intended that the pulse repetition rate is not a temporal cue for the patient (i.e., it should be sufficiently high so that the patient does not perceive tones with a frequency equal to the pulse repetition rate). The pulse repetition rate is usually chosen at greater than twice the bandwidth of the envelope signals (based on the Nyquist theorem).

In a CIS system, the stimulation pulses are applied in a strictly non-overlapping sequence. Thus, as a typical CIS-feature, only one electrode channel is active at a time and the overall stimulation rate is comparatively high. For example, assuming an overall stimulation rate of 18 kpps and a 12 channel filter bank, the stimulation rate per channel is 1.5 kpps. Such a stimulation rate per channel usually is sufficient for adequate temporal representation of the envelope signal. The maximum overall stimulation rate is limited by the minimum phase duration per pulse. The phase duration cannot be arbitrarily short because, the shorter the pulses, the higher the current amplitudes have to be to elicit action potentials in neurons, and current amplitudes are limited for various practical reasons. For an overall stimulation rate of 18 kpps, the phase duration is 27 $\mu$s, which is near the lower limit.

The Fine Structure Processing (FSP) strategy by Med-El uses CIS in higher frequency channels, and uses fine structure information present in the band pass signals in the lower frequency, more apical electrode channels. In the FSP electrode channels, the zero crossings of the band pass filtered time signals are tracked, and at each negative to positive zero crossing, a Channel Specific Sampling Sequence (CSSS) is started. Typically CSSS sequences are applied on up to 3 of the most apical electrode channels, covering the frequency range up to 200 or 330 Hz. The FSP arrangement is described further in Hochmair I, Nopp P, Jolly C, Schmidt M, Schößer H, Garnham C, Anderson I, MED-EL Cochlear Implants: State of the Art and a Glimpse into the Future, Trends in Amplification, vol. 10, 201-219, 2006. The FS4 coding strategy differs from FSP in that up to 4 apical channels can have their fine structure information used. In FS4-p, stimulation pulse sequences can be delivered in parallel on any 2 of the 4 FSP electrode channels. With the FSP and FS4 coding strategies, the fine structure information is the instantaneous frequency information of a given electrode channel, which may provide users with an improved hearing sensation, better speech understanding and enhanced perceptual audio quality. See, e.g., U.S. Patent 7,561,709; Lorens et al. "Fine structure processing improves speech perception as well as objective and subjective benefits in pediatric MED-EL COMBI 40+ users." International journal of pediatric otorhinolaryngology 74.12 (2010): 1372-1378; and Vermeire et al., "Better speech recognition in noise with the fine structure processing coding strategy." ORL 72.6 (2010): 305-311.

Many cochlear implant coding strategies use what is referred to as an n-of-m approach where only some number n electrode channels with the greatest amplitude are stimulated in a given sampling time frame. If, for a given time frame, the amplitude of a specific electrode channel remains higher than the amplitudes of other channels, then that channel will be selected for the whole time frame. Subsequently, the number of electrode channels that are available for coding information is reduced by one, which results in a clustering of stimulation pulses. Thus, fewer electrode channels are available for coding important temporal and spectral properties of the sound signal such as speech onset.

In addition to the specific processing and coding approaches discussed above, different specific pulse stimulation modes are possible to deliver the stimulation pulses with specific electrodes -*i.e.* mono-polar, bi-polar, tri-polar, multi-polar, and phased-array stimulation. And there also are different stimulation pulse shapes-*i.e.* biphasic, symmetric triphasic, asymmetric triphasic pulses, or asymmetric pulse shapes. These various pulse stimulation modes and pulse shapes each provide different benefits; for example, higher tonotopic selectivity, smaller electrical thresholds, higher electric dynamic range, less unwanted side-effects such as facial nerve stimulation, etc.

Fine structure coding strategies such as FSP and FS4 use the zero-crossings of the band-pass signals to start a channel-specific sampling sequence (CSSS) pulse sequences for delivery to the corresponding electrode contact. Zero-crossings reflect the dominant instantaneous frequency quite robustly in the absence of other spectral components. But in the presence of higher harmonics and noise, problems can arise. See, e.g., WO 2010/085477 and Gerhard, David, Pitch extraction and fundamental frequency: History and current techniques, Regina: Department of Computer Science, University of Regina, 2003.

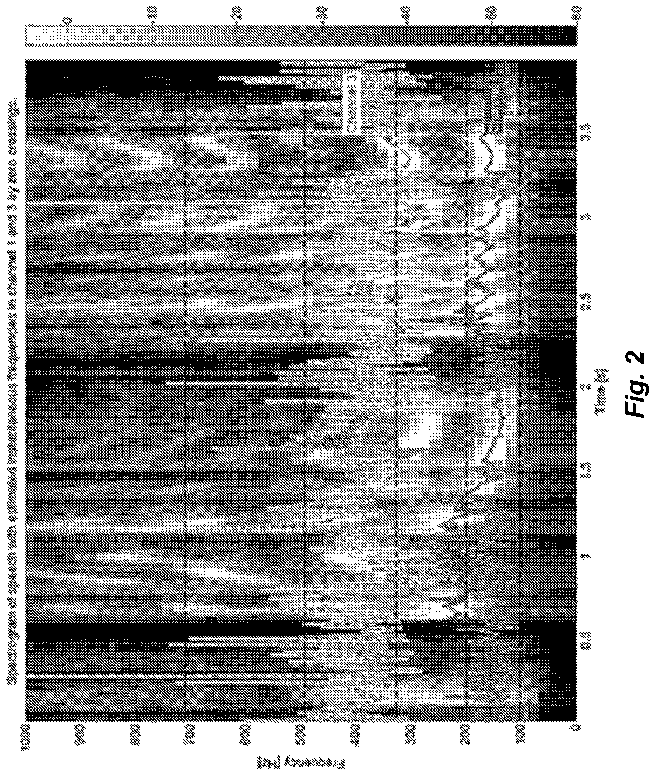

Figure 2 shows an example of a spectrogram for a sample of clean speech including estimated instantaneous frequencies for Channels 1 and 3 as reflected by evaluating the signal zero-crossings, indicated by the vertical dashed lines. The horizontal black dashed lines show the channel frequency boundaries-Channels 1, 2, 3 and 4 range between 100, 198, 325, 491 and 710 Hz, respectively. It can be seen in Figure 2 that during periods of a single dominant harmonic in a given frequency channel, the estimate of the instantaneous frequency is smooth and robust; for example, in Channel 1 from 1.6 to 1.9 seconds, or in Channel 3 from 3.4 to 3.5 seconds. When additional frequency harmonics are present in a given channel, or when the channel signal intensity is low, the instantaneous frequency estimation becomes inaccurate, and, in particular, the estimated instantaneous frequency may even leave the frequency range of the channel.

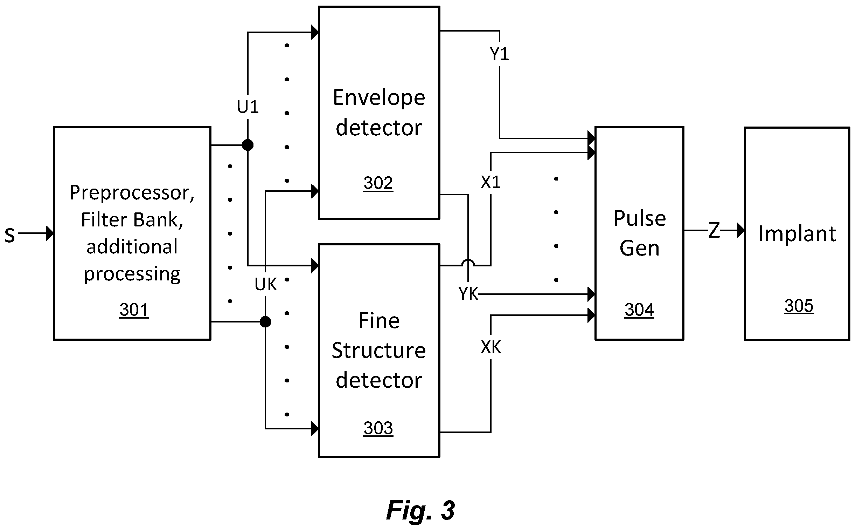

Figure 3 shows various functional blocks in a signal processing arrangement for a typical hearing implant. The initial input sound signal is produced by one or more sensing microphones, which may be omnidirectional and/or directional. Preprocessor Filter Bank **301** pre-processes this input sound signal with a bank of multiple parallel band pass filters (e.g. Infinite Impulse Response (IIR) or Finite Impulse Response(FIR)), each of which is associated with a specific band of audio frequencies; for example, using a filter bank with 12 digital Butterworth band pass filters of 6th order, Infinite Impulse Response (IIR) type, so that the acoustic audio signal is filtered into some K band pass signals, $U_1$ to $U_K$ where each signal corresponds to the band of frequencies for one of the band pass filters. Each output of sufficiently narrow CIS band pass filters for a voiced speech input signal may roughly be regarded as a sinusoid at the center frequency of the band pass filter which is modulated by the envelope signal. This is also due to the quality factor ($Q \approx$ 3) of the filters. In case of a voiced speech segment, this envelope is approximately periodic, and the repetition rate is equal to the pitch frequency. Alternatively and without limitation, the Preprocessor Filter Bank **301** may be implemented based on use of a fast Fourier transform (FFT) or a short-time Fourier transform (STFT). Based on the tonotopic organization of the cochlea, each electrode contact in the scala tympani typically is associated with a specific band pass filter of the Preprocessor Filter Bank 301. The Preprocessor Filter Bank 301 also may perform other initial signal processing functions such as and without limitation automatic gain control (AGC) and/or noise reduction and/or wind noise reduction and/or beamforming and other well-known signal enhancement functions.

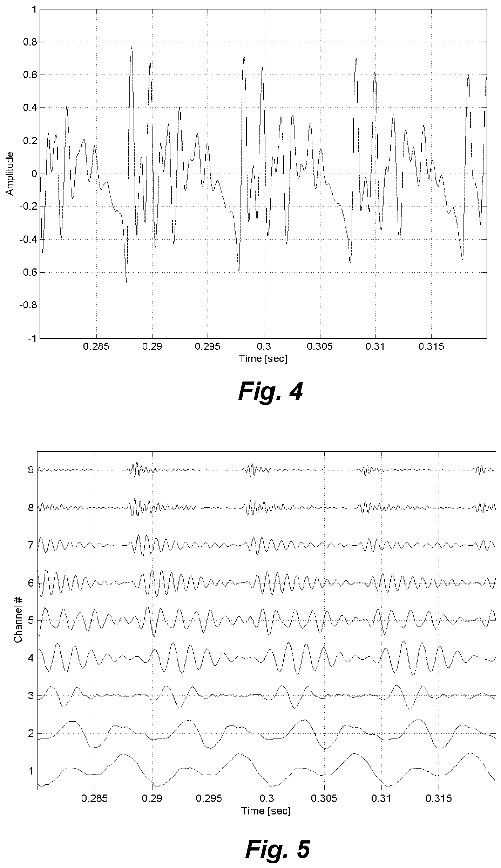

Figure 4 shows an example of a short time period of an input speech signal from a sensing microphone, and Figure 5 shows the microphone signal decomposed by band-pass filtering by a bank of filters. An example of pseudocode for an infinite impulse response (IIR) filter bank based on a direct form II transposed structure is given by Fontaine et al., Brian Hears: Online Auditory Processing Using Vectorization Over Channels, Frontiers in Neuroinformatics, 2011.

The band pass signals $U_1$ to $U_K$ (which can also be thought of as electrode channels) are output to an Envelope Detector **302** and Fine Structure Detector **303**. The Envelope Detector **302** extracts characteristic envelope signals outputs $Y_1, ..., Y_K$ that represent the channel-specific band pass envelopes. The envelope extraction can be represented by $Y_k = LP(|U_k|)$, where |.| denotes the absolute value and LP(.) is a low-pass filter; for example, using 12 rectifiers and 12 digital Butterworth low pass filters of 2nd order, IIR-type. Alternatively, the Envelope Detector **302** may extract the Hilbert envelope, if the band pass signals $U_1, ..., U_K$ are generated by orthogonal filters.

The Fine Structure Detector **303** functions to obtain smooth and robust estimates of the instantaneous frequencies in the signal channels, processing selected temporal fine structure features of the band pass signals $U_1, ..., U_K$ to generate stimulation timing signals $X_1, ..., X_K$. In the following discussion, the band pass signals $U_1, ..., U_k$ are assumed to be real valued signals, so in the specific case of an analytic orthogonal filter bank, the Fine Structure Detector **303** considers only the real valued part of $U_k$. The Fine Structure Detector **303** is formed of K independent, equally-structured parallel sub-modules.

The extracted band-pass signal envelopes $Y_1, ..., Y_K$ from the Envelope Detector **302,** and the stimulation timing signals $X_1, ..., X_K$ from the Fine Structure Detector **303** are input signals to a Pulse Generator **304** that produces the electrode stimulation signals Z for the electrode contacts in the implanted electrode array **305**. The Pulse Generator **304** applies a patient-specific mapping function-for example, using instantaneous nonlinear compression of the envelope signal (map law)-That is adapted to the needs of the individual cochlear implant user during fitting of the implant in order to achieve natural loudness growth. The Pulse Generator **304** may apply logarithmic function with a form-factor C as a loudness mapping function, which typically is identical across all the band pass analysis channels. In different systems, different specific loudness mapping functions other than a logarithmic function may be used, with just one identical function is applied to all channels or one individual function for each channel to produce the electrode stimulation signals. The electrode stimulation signals typically are a set of symmetrical biphasic current pulses.

CN108231067A discloses sound scenery recognition methods based on convolutional neural networks and random forest classification. First, sound scenery generates Mel energy spectrums and its segment sample set by Mel wave filters. Then, two benches training is carried out to CNN using segment sample set, blocks the feature output of full articulamentum, and obtain the CNN features of segment sample set. Finally, classified with random forest to the CNN features of segment sample set, a final recognition result is obtained.

Deliang Wang: "Deep Learning Reinvents the Hearing Aid" IEEE Spectrum, 1 December 2016 (2016-12-01), pages 1-8, describes training a deep neural network to use attributes to distinguish speech from noise. This training occurred in two phases: First, the program's parameters were set through unsupervised learning. Then samples of noisy speech and their corresponding results were used on the ideal binary mask to complete the second phase of training, which was the supervised learning. The error of the neural network, measured as the discrepancy between the ideal binary mask and the result at the neural network's final laver (output layer) was calculated. This error was used to change the weights of the neural network's connections. One refinement along the way was to build a second deep neural network that would be fed by the first one and fine-tune its results. While that first network had focused on labeling attributes within each individual time-frequency unit, the second network would examine the attributes of several units near a particular one.

## SUMMARY OF THE INVENTION

The present invention is set out in the appended claims. Embodiments of the present invention are directed to a signal processing system and method to generate stimulation signals for a hearing implant implanted in a patient. An audio scene classifier is configured for classifying an audio input signal from an audio scene and includes a pre-processing neural network configured for pre-processing the audio input signal based on initial classification parameters to produce an initial signal classification, and a scene classifier neural network configured for processing the initial scene classification based on scene classification parameters to produce an audio scene classification output. The initial classification parameters reflect neural network training based on a first set of initial audio training data, and the scene classification parameters reflect neural network training on a second set of classification audio training data separate and different from the first set of initial audio training data. The pre-processing neural network optimizes meta-parameters without explicit training of weights via back propagation, and the classifier neural network includes a back propagation procedure. A hearing implant signal processor configured for processing the audio input signal and the audio scene classification output to generate the stimulation signals to the hearing implant for perception by the patient as sound.

In further specific embodiments, the pre-processing neural network includes successive recurrent convolutional layers, which may be implemented as recursive filter banks. The pre-processing neural network may include an envelope processing block configured for calculating sub-band signal envelopes for the audio input signal. The pre-processing neural network also may include a pooling layer configured for signal decimation within the pre-processing neural network. The initial signal classification may be a multi-dimensional feature vector. The scene classifier neural network may be a fully connected neural network layer or a linear discriminant analysis (LDA) classifier.

BRIEF DESCRIPTION OF THE DRAWINGS

Figure 1 shows the anatomy of a typical human ear and components in a cochlear implant system.

Figure 2 shows an example spectrogram of a speech sample.

Figure 3 shows major signal processing blocks of a typical cochlear implant system.

Figure 4 shows an example of a short time period of an input speech signal from a sensing microphone.

Figure 5 shows the microphone signal decomposed by band-pass filtering by a bank of filters.

Figure 6 shows major functional blocks in a signal processing system according to an embodiment of the present invention.

Figure 7 shows processing steps in initially training a pre-processing neural network according to an embodiment of the present invention.

Figure 8 shows processing steps in iteratively training a classifier neural network according to an embodiment of the present invention.

Figure 9 shows functional details of a pre-processing neural network according to one specific embodiment of the present invention.

Figure 10 shows an example of how filter bank filter bandwidths may be structured according to an embodiment of the present invention.

DETAILED DESCRIPTION OF SPECIFIC EMBODIMENTS

Neural network training is a complicated and demanding process that requires a lot of training data for optimizing the parameters of the network. The effectiveness of the training further very much depends on the training data that is used. Many undesirable side effects may occur after the training, and it might even happen that the neural network does not even perform the intended task. This problem is particularly pronounced when trying to classify audio scenes for hearing implants where a nearly infinite number of variations exist for each classified scene and seamless transitions occur between distinct scenes.

Embodiments of the present invention are directed to an audio scene classifier for hearing implants that uses a multi-layer neural network optimized for iterative training of a low number of parameters that can be trained with reasonable effort and sized training sets. This is accomplished by separating the neural network into an initial pre-processing neural network whose output is then input to a classification neural network. This allows for separate training of the individual neural networks and thereby allows use of smaller training sets and faster training that is carried out in a two-step process as described below.

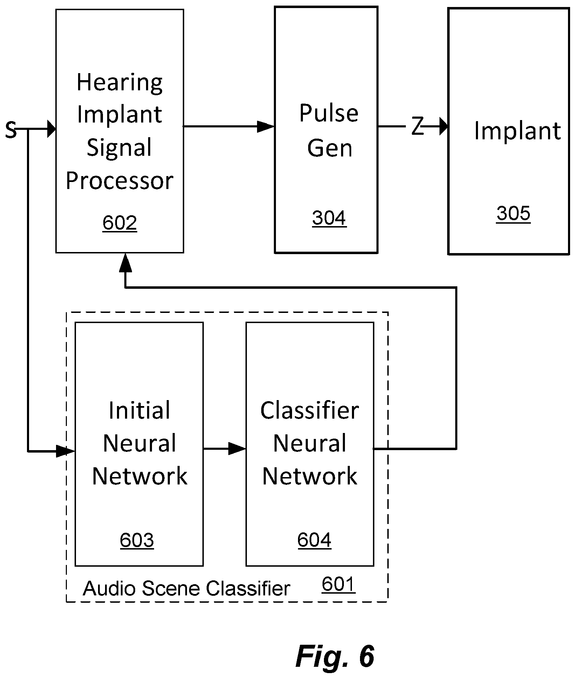

Figure 6 shows major functional blocks in a signal processing system according to an embodiment of the present invention for generating stimulation signals for a hearing implant implanted in a patient. An audio scene classifier **601** is configured for classifying an audio input signal from an audio scene and includes a pre-processing neural network **603** that is configured for pre-processing the audio input signal based on initial classification parameters to produce an initial signal classification, and a scene classifier neural network **604** that is configured for processing the initial scene classification based on scene classification parameters to produce an audio scene classification output. The initial classification parameters reflect neural network training based on a first set of initial audio training data, and the scene classification parameters reflect neural network training on a second set of classification audio training data separate and different from the first set of initial audio training data. A hearing implant signal processor **602** is configured for processing the audio input signal and the output of the audio scene classifier **601** to generate the stimulation signals to a pulse generator **304** to provide to the hearing implant **305** for perception by the patient as sound.

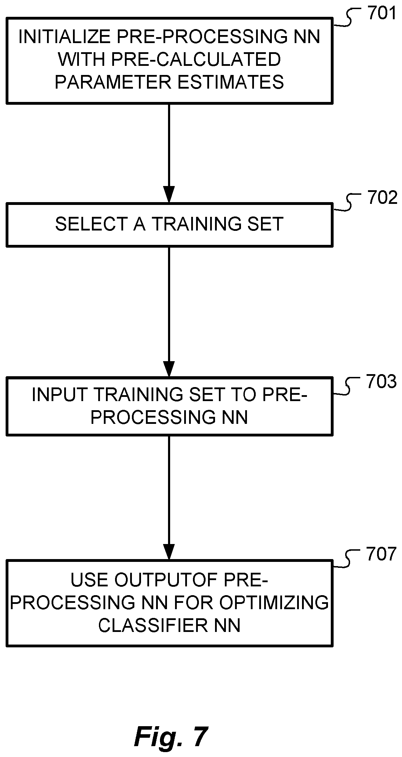

Figure 7 shows processing steps in initially training the pre-processing neural network **603,** which starts, step **701,** by initializing the pre-processing neural network **603** with precalculated parameter that are within an expected range of parameters, for example, in the middle of a parameter range. A first training set of audio training data (Training Set 1) is selected, step **702,** and input for training of the pre-processing neural network **603**, step **703**. The output from the pre-processing neural network **603** then, step **704,** is used as the input to the classifier neural network **604** for optimizing it using various known optimization methods.

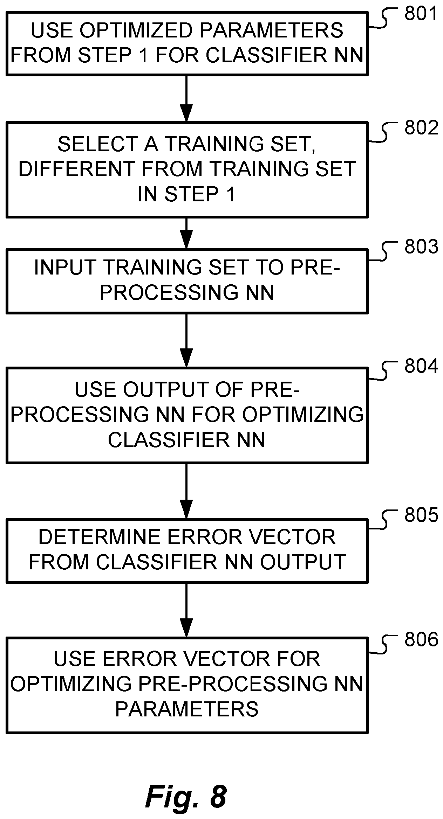

Figure 8 then shows various subsequent processing steps in iteratively training a classifier neural network **604** starting with the optimized parameters from the initial training of the pre-processing neural network as discussed above with regards to Figure 7, step **801**. A second training set of audio training data (Training Set 2), which is different from the first training set, is selected, step **802,** and input to the pre-processing neural network **603**. The output from the pre-processing neural network **603** is further input and processed by the classification neural network **604**, step **804**. An error vector then is calculated, step **805,** by comparing the output from the classification neural network **604** to the audio scene that the second training set data should belong to. The error vector then, step **806,** is used to optimize the pre-processing neural network **603**. The new parameterization of the pre-processing neural network **603,** then leads to a two-step iterative training procedure that ends when selected stopping criteria are met.

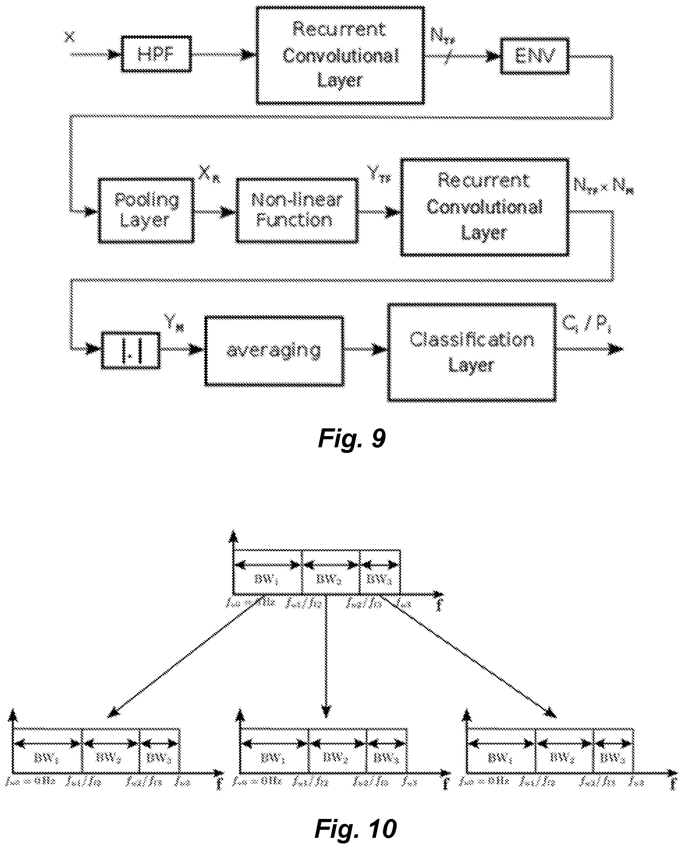

Figure 9 shows functional details of a pre-processing neural network according to one specific embodiment of the present invention with several linear and non-linear processing blocks. In the specific example shown, there are two successive recurrent convolutional layers, pooling layers, non-linear functions and an averaging layer. The recurrent convolutional layers can be implemented as recursive filters banks. Without loss of generality, the input signal is assumed to be an audio signal $x(k)$ with length $N$, which is first high-pass filtered (HPF-block) and then fed into $N_{TF}$ parallel processing blocks that act as band pass filters. This leads to $N_{TF}$ output sub band signals $x_{T,i}(k)$ with different spectral contents. The band pass filtered sub band signals can be expressed by the equation:

$$x_{T,i}(k) = \sum_{n=0}^{P_1} b_{i,n} x(k-n) - \sum_{m=1}^{P_2} a_{i,m} x_{T,i}(k-m)$$

where $b_{i,n}$ are the feed forward coefficients, and $a_{i,n}$ the feedback coefficients of the i-th filter block. The filter order is $P = max(P_1, P_2)$.

The sub band signals envelopes then are calculated by rectification and low pass filtering. Note that any other method for determining the envelopes can be used, too. The low pass filter may be, for example, a fifth-order recursive Chebyshev II filter with 30 dB attenuation in the stop band. The cutoff frequency $f_{T,s}$ can be determined by the highest band pass filter upper edge frequency of the next filter bank plus an additional offset. The low pass filter prior to the pooling layer (decimation block) helps to avoid aliasing effects. The output of the pooling layer is the subsampled sub band envelope signal $x_{R,i}(n)$, which then is processed through the non-linear function block. This non-linear function can include, for example, range limitation, normalization and further non-linear functions such as logarithms or exponentials.

The output $\mathbf{Y_{TF}}$ of this stage is a $N_{TF} \times N_R$ matrix with $N_R = \lfloor \frac{N}{R} \rfloor$ where $R$ is a decimation factor and $\lfloor \cdot \rfloor$ is the floor operation.

The output signals $y_{R,i} = [y_{R,i}(1) \quad y_{R,i}(2) \dots \quad y_{R,i}(N_R)]$ are arranged into a matrix $\mathbf{Y}_{TF} = \begin{bmatrix} \mathbf{y}_{R,1}^T \ \mathbf{y}_{R,2}^T \ \cdots \ \mathbf{y}_{R,N_{TF}}^T \end{bmatrix}^T$ where each row corresponds to a specific frequency band. The output of this layer $Y_{TF}$ (where each row corresponds to a specific frequency band) is first fed row by row to $N_M$ recurrent convolutional layers which can represent a bank of modulation filters. The modulation filters can be individually parameterized for each frequency band yielding an overall number of filters $N_M \times N_{TF}$. The ordering of the parallel band pass filters for each frequency is analogous to the parallel band pass. The absolute values of the filtered signals $\hat{x}_{M,i}(n)$ with i $\in \{1,...N_{TF}, \times N_M\}$ of these filter banks $y_{M,i}(n) = |\hat{x}_{M,i}(n)|$ are averaged and the final result is a feature vector $Y_{MF}$ with dimensions $N_{TF} \times N_M$. This feature vector is the output of the pre-processing neural network and input to the classification neural network.

The classification neural network may be for example a fully connected neural network layer, a linear discriminant analysis (LDA) classifier, or a more complex classification layer. The outputs of this layer are the predefined class labels $C_i$ or/and probabilities $P_i$ for them.

As explained above, the multi-layer neural network arrangement is iteratively optimized. First an initial setting for the pre-processing neural network is chosen and the feature vectors $Y_{MF}$ for the Training Set 1 are calculated. For this feature vector, the classification neural network can be trained by a standard method such as back propagation or LDA. Then for Training Set 2, the corresponding class labels or/and probabilities are calculated and used to calculate an error vector that is input to the training approach of the pre-processing neural network. This yields a new setting for the pre-processing neural network. With this new setting, the next iteration of the training procedure starts.

The training of the pre-processing neural network optimizes it in the sense of minimizing an error function, minimizing the mismatch between the estimated class labels and the ground truth class labels. Instead of explicitly training the weights of the pre-processing neural network via a back propagation procedure (which is the state-of-the art algorithm for training neural networks), meta-parameters are optimized, for example with generic algorithms or model-based optimization approaches. This significantly reduces the number of tunable weights and also reduces the amount of training data needed due to lower weight vector dimensionality. As a result the neural network has better generalization capabilities, which are important for its performance in previously unseen conditions.

The meta-parameters could be, for example, filter bandwidths and the neural network weights would be the coefficients of the corresponding filters. In this example, any filter design rule can be applied for computing the filter coefficients. However, other rules for mapping meta-parameters to network weights may be used as well. This mapping could be learned automatically via an optimization procedure and/or may be adaptive such that the network weights are updated during optimization and/or during the operation of the trained network. The optimal bandwidths of the filter for a given classification problem can be found by known optimization algorithms. Before running the optimization process, a filter design rule is chosen for mapping meta-parameters to filter coefficients. For example, Butterworth filters can be chosen for the first filter bank and Chebychev 2 filters for the second one, or vice versa.

Figure 10 shows an example of how filter bank filter bandwidths may be structured according to an embodiment of the present invention. The first filters in the filter banks are low pass filters where the edge frequency is the lower edge frequency of the successive band pass filter and so on. This mapping rule from meta-parameters to network weights ensures that the network uses all information available in the input signal. The specification of the network structure via meta-parameters and filter design rules reduces the optimization complexity. The upper and lower edge frequencies of each filter can also be indeendently trained and other design rules are possible. With this approach, the initialization of the pre-processing neural network can be done by selection of all boundary frequencies according to $0 = f_{u_0} < f_{u_1} =$

$$f_{l_2} < \cdots < f_{l_N} \leq \frac{fs}{2}$$, where $fs$ is sampling frequency of corresponding input signal. The network weights can be achieved by using the defined mapping rule.

As mentioned above, there are $N_{TF} \cdot (N_M + 1) - 1$ independently tunable parameters. Finding optimal parameters using an exhaustive search may not be feasible due to the high dimensionality. A gradient descent algorithm also may not be suitable because the multimodal cost function (classification error) is not differentiable. Thus a Covariance Matrix Adaptation Evolution Strategy (CMA-ES) can be used based in order to find an ideal parameter set for the feature extraction step (see e.g., N. Hansen, "The CMA evolution strategy: A comparing review," in Towards a new evolutionary computation. Advances in estimation of distribution algorithms. Springer, 2006, pp. 75-102, which is incorporated herein by reference in its entirety). ES is a subclass of evolutionary algorithms (EA) and shares the idea of imitating natural evolution, for instance by mutation and selection, and it does not require the computation of any derivatives (H. Beyer, Theory of Evolution Strategies, Springer, 2001 edition; incorporated herein by reference in its entirety). The optimal parameter set can be iteratively approximated by evaluating a fitness function after each step, where the fitness function or cost function may be the classification error (the ratio of the number of misclassified objects to the number of all objects) of the LDA classifier as a function of the independently tunable parameters.

The basic equation for CMA-ES is the sampling equation of new search points (Hansen 2006):

$$\mathbf{x}_k^{g+1} \sim \mathbf{m}^{(g)} + \sigma^{(g)} \mathcal{N}\left(\mathbf{0}, \mathbf{C}^{(g)}\right) \quad \text{for } k = 1, \ldots, \lambda$$

where $g$ is the index of the current generation (iteration), $\mathbf{x}_k^{g+1}$ is the $k$-th offspring from generation $g + 1$, $\lambda$ is the number of offspring, $\mathbf{m}^{(g)}$ is the mean value of the search distribution at generation $g$, $\mathcal{N}(0, \mathbf{C}^{(g)})$ is a multivariate normal distribution with the covariance matrix $\mathbf{C}^{(g)}$ of generation $g$, and $\sigma^{(g)}$ is the step-size of generation $g$. From the $\lambda$ sampled new solution candidates, the $\mu$ best points (in terms of minimal cost function) are selected and the new mean of generation $g + 1$ is determined by a weighted average according to:

$$\mathbf{m}^{(g+1)} = \sum_{i=1}^{\mu} w_i \mathbf{x}_{i:\mu}^{g+1}$$

$$\sum_{i=1}^{\mu} w_i = 1, \qquad w_1 \geq w_2 \geq \cdots \geq w_\mu > 0$$

In each iteration of the CMA-ES, the covariance matrix **C** and the step-size $\sigma$ are adapted according to the success of the sampled offspring. The shape of the multivariate normal distribution is formed in the direction of the old mean $\mathbf{m}^{(g)}$ towards the new mean $\mathbf{m}^{(g+1)}$. The sampling, selection and recombination steps are repeated until reaching either a predefined threshold on the cost function or a maximum number of generations, or the range of the current functional evaluation is below a threshold (local minima is reached). The allowed search space of the parameters can be restricted to intervals as described by Colutto et al. in S. Colutto, F. Frühauf, M. Fuchs, and O. Scherzer, "The CMA-ES on Riemannian manifolds to reconstruct shapes in 3-D voxel images," IEEE Transactions on Evolutionary Computation, vol. 14, no. 2, pp. 227-245, April 2010, which is incorporated herein by reference in its entirety. For a more detailed description of CMA-ES, in particular on how the covariance matrix c and the step-size $\sigma$ are adapted in each step, as well as a Matlab implementation, please refer to Hansen 2006. Other generic algorithms such as particle swarm optimization also can be used.

Optimizing the filter bank parameters used for deriving the weights of the network in order to decrease the classification error is a challenging task due to its high dimensionality and multi-modal error function. Brute-force and gradient-descent may not be feasible for this task. One useful approach may be based on Model-Based Optimization (MBO) (see Alexander Forrester, Andras Sobester, and Andy Keane. Engineering Design via Surrogate Modeling: A Practical Guide. Wiley, September 2008; and Claus Weihs, Swetlana Herbrandt, Nadja Bauer, Klaus Friedrichs, and Daniel Horn. Efficient Global Optimization: Motivation, Variations, and Applications. In ARCHIVES OF DATA SCIENCE, 2016).

MBO is an iterative approach used to optimize a black box objective function. It is used where the evaluation of an objective function (e.g., the classification error depending on different filter bank parameters) is expensive in terms of available resources such as computational time. An approximation model, a so-called surrogate model, is constructed of this expensive objective function in order to find the optimal parameter for a given problem. The evaluation of the surrogate model is cheaper than the original objective function. The MBO steps can be divided as follows:

- Design a sampling plan,
- Constructing a surrogate model,
- Exploring and exploiting a surrogate model.

A high dimensional multi-modal parameter space is assumed and the goal of the optimization is to find the point which minimizes the cost function. The initial step of the MBO is to construct a sampling plan. This means that n points are determined which will then be evaluated by the objective function. These n points should cover the whole region of the parameter space, and for this the space-filling design called Latin hypercube design can be used. The parameter space is divided into n equal-sized hyper-cubes (bins), where $n \in \{5k, 6k, ..., 10k\}$ is recommended and $k$ is the number of parameters. The points are then placed in the bins such that "from each occupied bin we could exit the parameter space along any direction parallel with any of the axes without encountering any other occupied bins" (Forrester 2008). Randomly set points do not guarantee the space-filling property of the sampling plan **X** (n x $k$ matrix) and to evaluate the space-fillingness of **X** the *maximin* metric of Morris and Mitchell is used:

"We call **X** the maximin plan among all available plans if it maximizes $d_1$, among plans for which this is true, minimizes $J_1$, among all plans for which this is true, maximizes $d_2$, among all plans for which this is true, minimizes $J_2$, ..., minimizes $J_m$."

With $d_1, d_2, d_3,..., d_m$ the list of unique values of distances between all possible pairs of points in the sampling plan **X** sorted in ascending order, and $J_j$ is the number of pairs of points in **X** separated by the distance $d_j$.

The above definition means that one sequentially maximizes $d_1$ and then minimizes $J_1$, maximizes $d_2$ and then minimizes $J_2$ and so on. Or in other words, the goal is to have as minimal distinct pairs with maximum distance as possible. As a metric for the distance d between two points the *p-norm* is used:

$$d_p(\mathbf{x}^{(i_1)}, \mathbf{x}^{(i_2)}) = \left( \sum_{j=1}^{k} |x_j^{i_1} - x_j^{i_2}|^p \right)^{1/p}$$

where $p = 1$ is used as the rectangular norm. Based on the above definition of a maximin plan, Morris and Mitchell propose comparing sampling plans according to the criterion:

$$\Phi_q(\mathbf{X}) = \left( \sum_{j=1}^{m} J_j d_j^{-q} \right)^{1/q}$$

The smaller $\Phi_q$, the better **X** fulfills the space-filling property (Forrester 2008). For the *best* Latin hypercube, Morris and Mitchell recommend minimizing $\Phi_q$ for q = 1, 2, 5, 10, 20, 50 and 100 and choosing the sampling plan with the smallest $\Phi_q$.

A surrogate model $\hat{g}(\mathbf{x})$ can be constructed such that it is a reasonable approximation of the unknown objective function $f(\mathbf{x})$ (where **x** is a $k$ dimensional vector pointing to a point in the parameter space). Different types of models can be constructed such as an ordinary Kriging model:

$$\hat{g}(\mathbf{x}) = \mu + Z(\mathbf{x})$$

where $\mu$ is a constant global mean and Z(x) is a Gaussian process. The mean of this Gaussian process is 0, and its covariance is:

$$\mathrm{Cov}(Z(\mathbf{x}), Z(\mathbf{x})) = \sigma^2 \rho(\mathbf{x} - \mathbf{x}', \Psi)$$

with $\rho$ the Matern 3/2 kernel function and $\psi$, a scaling parameter. The constant $\sigma^2$ is global variance. The Matern 3/2 kernel is defined as:

$$\rho(\mathbf{x} - \mathbf{x}') = \left( 1 + \sqrt{3} \frac{|\mathbf{x} - \mathbf{x}'|}{\Psi} \right) \exp \left( -\sqrt{3} \frac{|\mathbf{x} - \mathbf{x}'|}{\Psi} \right)$$

So the unknown parameters of this model are $\mu$, $\sigma^2$ and $\psi$, which are estimated by using the n previously by the objective function evaluated points $\mathbf{y} = (y_1, ..., y_n)^T$.

The likelihood function is:

$$L(\mu, \sigma^2, \Psi) = \frac{1}{\sqrt{(2\pi)^n \sigma^{2n} det(R)}} \exp \left( -\frac{1}{2\sigma^2} (\mathbf{y} - \mathbf{1}\mu)^T R^{-1} (\mathbf{y} - \mathbf{1}\mu) \right)$$

with $R(\psi) = (\rho(\mathbf{x}_i - \mathbf{x}_j, \psi))_{i,j=1,...,n}$ and $det(R)$ its determinant. From this the Maximum likelihood estimation of the unknown parameters can be determined:

$$\mu = \arg\max_{\mu} L(\mu, \sigma^2, \Psi)$$

$$\sigma^2 = \arg\max_{\sigma^2} L(\mu, \sigma^2, \Psi)$$

$$\Psi = \arg\max_{\psi} L(\mu, \sigma^2, \Psi)$$

The surrogate prediction $\hat{f}_n(\mathbf{x})$ and the corresponding prediction uncertainty $\hat{s}_n(\mathbf{x})$ (see Weihs 2016) can be determined based on the first n evaluations of f. The estimated surrogate function follows a normal distribution $\hat{g}(\mathbf{x}) \sim \mathcal{N}\left(\hat{f}_n(\mathbf{x}), \hat{s}_n^2(\mathbf{x})\right)$. With the actual best value $y_{\min} = \min\limits_{i=1,\dots,n} y_i = \min\limits_{i=1,\dots,n} f(\mathbf{x}_i)$, then the improvement for a point x and the estimated surrogate $\hat{g}(\mathbf{x})$ is $I_n(\mathbf{x}) = \max(y_{\min} - \hat{g}(\mathbf{x}), 0)$. The next point to evaluate is found by maximizing the expected improvement:

$$\mathbf{x}_{n+1} = \arg\max_{\mathbf{x}} \mathrm{E}\big(I_n(\mathbf{x})\big)$$

The above criterion gives a balance between exploration (improving global accuracy of the surrogate model) and exploitation (improving local accuracy in the region of the optimum of the surrogate model). This ensures that the optimizer will not get stuck in local optima and yet converges to an optimum. After each iteration of MBO, the surrogate model will be updated. Different convergence criteria could be chosen to determine when to stop evaluating new points for updating the surrogate model. Some criteria could be, e.g., to define a preset number of iterations and stop after this or to stop after the expected improvement drops below a predefined threshold.

The hearing implant may be, without limitation, a cochlear implant, in which the electrodes of a multichannel electrode array are positioned such that they are, for example, spatially divided within the cochlea. The cochlear implant may be partially implanted, and include, without limitation, an external speech/signal processor, microphone and/or coil, with an implanted stimulator and/or electrode array. In other embodiments, the cochlear implant may be a totally implanted cochlear implant. In further embodiments, the multi-channel electrode may be associated with a brainstem implant, such as an auditory brainstem implant (ABI).

Embodiments of the invention may be implemented in part in any conventional computer programming language. For example, preferred embodiments may be implemented in a procedural programming language (e.g., "C") or an object oriented programming language (e.g., "C++", Python). Alternative embodiments of the invention may be implemented as preprogrammed hardware elements, other related components, or as a combination of hardware and software components.

Embodiments can be implemented in part as a computer program product for use with a computer system. Such implementation may include a series of computer instructions fixed either on a tangible medium, such as a computer readable medium (e.g., a diskette, CD-ROM, ROM, or fixed disk) or transmittable to a computer system, via a modem or other interface device, such as a communications adapter connected to a network over a medium. The medium may be either a tangible medium (e.g., optical or analog communications lines) or a medium implemented with wireless techniques (e.g., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (e.g., shrink wrapped software), preloaded with a computer system (e.g., on system ROM or fixed disk), or distributed from a server or electronic bulletin board over the network (e.g., the Internet or World Wide Web). Of course, some embodiments of the invention may be implemented as a combination of both software (e.g., a computer program product) and hardware. Still other embodiments of the invention are implemented as entirely hardware, or entirely software (e.g., a computer program product).

The invention is set out in the claims that follow.

**Claims**

1. A signal processing method for generating stimulation signals for a hearing implant (305) implanted in a patient, the method comprising:

   classifying an audio input signal from an audio scene with a multi-layer neural network, the classifying comprising:

   a) pre-processing the audio input signal with a pre-processing neural network (603) using initial classification parameters to produce an initial signal classification, and
   b) processing the initial signal classification with a scene classifier neural network (604) using scene classification parameters to produce an audio scene classification output,

wherein the initial classification parameters reflect neural network training based on a first set of initial audio training data, and the scene classification parameters reflect neural network training on a second set of classification audio training data separate and different from the first set of initial audio training data, wherein the pre-processing neural network (603) optimizes meta-parameters without explicit training of weights via back propagation, and wherein the classifier neural network (604) includes a back propagation procedure;
processing the audio input signal and the audio scene classification output with a hearing implant signal processor (602) for generating the stimulation signals.

2. The method according to claim 1, wherein the pre-processing neural network includes successive recurrent convolutional layers.

3. The method according to claim 2, wherein the recurrent convolutional layers are implemented as recursive filter banks.

4. The method according to claim 1, wherein the pre-processing neural network includes an envelope processing block configured for calculating sub-band signal envelopes for the audio input signal.

5. The method according to claim 1, wherein the pre-processing neural network includes a pooling layer configured for signal decimation within the pre-processing neural network.

6. The method according to claim 1, wherein the initial signal classification is a multi-dimensional feature vector.

7. The method according to claim 1, wherein the scene classifier neural network comprises a fully connected neural network layer.

8. The system according to claim 1, wherein the scene classifier neural network comprises a linear discriminant analysis (LDA) classifier.

9. A signal processing system for generating stimulation signals for a hearing implant (305) implanted in a patient, the system comprising:

an audio scene classifier (601) comprising a multi-layer neural network configured for classifying an audio input signal from an audio scene, wherein the audio scene classifier includes:

a) a pre-processing neural network (603) configured for pre-processing the audio input signal based on initial classification parameters to produce an initial signal classification, and
b) a scene classifier neural network (604) configured for processing the initial signal classification based on scene classification parameters to produce an audio scene classification output,

wherein the initial classification parameters reflect neural network training based on a first set of initial audio training data, and the scene classification parameters reflect neural network training on a second set of classification audio training data separate and different from the first set of initial audio training data, wherein the pre-processing neural network (603) optimizes meta-parameters without explicit training of weights via back propagation, and wherein the classifier neural network (604) includes a back propagation procedure;
a hearing implant signal processor (602) configured for processing the audio input signal and
the audio scene classification output for generating the stimulation signals.

10. The system according to claim 9, wherein the pre-processing neural network includes successive recurrent convolutional layers.

11. The system according to claim 10, wherein the recurrent convolutional layers are implemented as recursive filter banks.

12. The system according to claim 9, wherein the pre-processing neural network includes an envelope processing block configured for calculating sub-band signal envelopes for the audio input signal.

13. The system according to claim 9, wherein the pre-processing neural network includes a pooling layer configured for signal decimation within the pre-processing neural network.

14. The system according to claim 9, wherein the initial signal classification is a multi-dimensional feature vector.

15. The system according to claim 9, wherein the scene classifier neural network comprises a fully connected neural network layer or
wherein the scene classifier neural network comprises a linear discriminant analysis (LDA) classifier.

**Patentansprüche**

1. Signalverarbeitungsverfahren zum Erzeugen von Stimulationssignalen für ein Hörimplantat (305), das in einen Patienten implantiert ist, wobei das Verfahren umfasst:

   Klassifizieren eines Audioeingangssignals aus einer Audioszene mit einem mehrschichtigen neuronalen Netzwerk, wobei das Klassifizieren umfasst:

   a) Vorverarbeiten des Audioeingangssignals mit einem vorverarbeitenden neuronalen Netzwerk (603) unter Verwendung von anfänglichen Klassifizierungsparametern, um eine anfängliche Signalklassifizierung zu erzeugen, und
   b) Verarbeiten der anfänglichen Signalklassifizierung mit einem neuronalen Szenenklassifizierernetzwerk (604) unter Verwendung von Szenenklassifizierungsparametern, um eine Audioszenenklassifizierungsausgabe zu erzeugen,

   wobei die anfänglichen Klassifizierungsparameter ein neuronales Netzwerktraining basierend auf einem ersten Satz von anfänglichen Audiotrainingsdaten reflektieren und die Szenenklassifizierungsparameter ein neuronales Netzwerktraining auf einem zweiten Satz von Klassifizierungsaudiotrainingsdaten reflektieren, der von dem ersten Satz von anfänglichen Audiotrainingsdaten getrennt ist und sich davon unterscheidet, wobei das vorverarbeitende neuronale Netzwerk (603) Metaparameter ohne explizites Training von Gewichten über Rückausbreitung optimiert und wobei das neuronale Klassifiziererernetzwerk (604) ein Rückausbreitungsverfahren beinhaltet;
   Verarbeiten des Audioeingangssignals und der Audioszenenklassifizierungsausgabe mit einem Hörimplantatsignalprozessor (602) zum Erzeugen der Stimulationssignale.

2. Verfahren nach Anspruch 1, wobei das vorverarbeitende neuronale Netzwerk aufeinanderfolgende rekurrente Faltungsschichten beinhaltet.

3. Verfahren nach Anspruch 2, wobei die rekurrenten Faltungsschichten als rekursive Filterbänke implementiert sind.

4. Verfahren nach Anspruch 1, wobei das vorverarbeitende neuronale Netzwerk einen Hüllkurvenverarbeitungsblock beinhaltet, der zum Berechnen von Teilbandsignalhüllkurven für das Audioeingangssignal konfiguriert ist.

5. Verfahren nach Anspruch 1, wobei das vorverarbeitende neuronale Netzwerk eine Pooling-Schicht beinhaltet, die zur Signaldezimierung innerhalb des vorverarbeitenden neuronalen Netzwerks konfiguriert ist.

6. Verfahren nach Anspruch 1, wobei die anfängliche Signalklassifizierung ein mehrdimensionaler Merkmalsvektor ist.

7. Verfahren nach Anspruch 1, wobei das neuronale Netzwerk des Szenenklassifizierers eine vollständig verbundene neuronale Netzwerkschicht umfasst.

8. System nach Anspruch 1, wobei das neuronale Netzwerk des Szenenklassifizierers einen linearen Diskriminanzanalyse(LDA)-Klassifizierer umfasst.

9. Signalverarbeitungssystem zum Erzeugen von Stimulationssignalen für ein Hörimplantat (305), das in einen Patienten implantiert ist, wobei das System umfasst:

   einen Audioszenenklassifizierer (601), der ein mehrschichtiges neuronales Netzwerk umfasst, das zum Klassifizieren eines Audioeingangssignals aus einer Audioszene konfiguriert ist, wobei der Audioszenenklassifizierer beinhaltet:

a) ein vorverarbeitendes neuronales Netzwerk (603), das zum Vorverarbeiten des Audioeingangssignals basierend auf anfänglichen Klassifizierungsparametern konfiguriert ist, um eine anfängliche Signalklassifizierung zu erzeugen, und

b) ein neuronales Szenenklassifizierernetzwerk (604), das zum Verarbeiten der anfänglichen Signalklassifizierung basierend auf Szenenklassifizierungsparametern konfiguriert ist, um eine Audioszenenklassifizierungsausgabe zu erzeugen,

wobei die anfänglichen Klassifizierungsparameter ein neuronales Netzwerktraining basierend auf einem ersten Satz von anfänglichen Audiotrainingsdaten reflektieren und die Szenenklassifizierungsparameter ein neuronales Netzwerktraining auf einem zweiten Satz von Klassifizierungsaudiotrainingsdaten reflektieren, der von dem ersten Satz von anfänglichen Audiotrainingsdaten getrennt ist und sich davon unterscheidet, wobei das vorverarbeitende neuronale Netzwerk (603) Metaparameter ohne explizites Training von Gewichten über Rückausbreitung optimiert und wobei das neuronale Klassifizierernetzwerk (604) ein Rückausbreitungsverfahren beinhaltet;

einen Hörimplantatsignalprozessor (602), der zum Verarbeiten des Audioeingangssignals und der Audioszenenklassifizierungsausgabe zum Erzeugen der Stimulationssignale konfiguriert ist.

10. System nach Anspruch 9, wobei das vorverarbeitende neuronale Netzwerk aufeinanderfolgende rekurrente Faltungsschichten beinhaltet.

11. System nach Anspruch 10, wobei die rekurrenten Faltungsschichten als rekursive Filterbänke implementiert sind.

12. System nach Anspruch 9, wobei das vorverarbeitende neuronale Netzwerk einen Hüllkurvenverarbeitungsblock beinhaltet, der zum Berechnen von Teilbandsignalhüllkurven für das Audioeingangssignal konfiguriert ist.

13. System nach Anspruch 9, wobei das vorverarbeitende neuronale Netzwerk eine Pooling-Schicht beinhaltet, die zur Signaldezimierung innerhalb des vorverarbeitenden neuronalen Netzwerks konfiguriert ist.

14. System nach Anspruch 9, wobei die anfängliche Signalklassifizierung ein mehrdimensionaler Merkmalsvektor ist.

15. System nach Anspruch 9, wobei das neuronale Netzwerk des Szenenklassifizierers eine vollständig verbundene neuronale Netzwerkschicht umfasst oder
wobei das neuronale Netzwerk des Szenenklassifizierers einen linearen Diskriminanzanalyse(LDA)-Klassifizierer umfasst.

## Revendications

1. Procédé de traitement de signal pour générer des signaux de stimulation pour un implant auditif (305) implanté chez un patient, le procédé comprenant :

la classification d'un signal d'entrée audio provenant d'une scène audio à l'aide d'un réseau neuronal multicouche, la classification comprenant :

a) le prétraitement du signal d'entrée audio avec un réseau neuronal de prétraitement (603) en utilisant des paramètres de classification initiaux pour produire une classification initiale du signal, et

b) le traitement de la classification initiale du signal à l'aide d'un réseau neuronal classificateur de scène (604) en utilisant des paramètres de classification de scène pour produire une sortie de classification de scène audio,

dans lequel les paramètres de classification initiaux reflètent l'entraînement du réseau neuronal sur la base d'un premier ensemble de données d'entraînement audio initiales, et les paramètres de classification de scène reflètent l'entraînement du réseau neuronal sur un deuxième ensemble de données d'entraînement audio de classification distinctes et différentes du premier ensemble de données d'entraînement audio initiales, dans lequel le réseau neuronal de prétraitement (603) optimise les méta-paramètres sans entraînement explicite des poids par rétropropagation, et dans lequel le réseau neuronal classificateur (604) comprend une procédure de rétropropagation ;

le traitement du signal d'entrée audio et de la sortie de classification de scène audio à l'aide d'un processeur de signal d'implant auditif (602) pour générer les signaux de stimulation.

2. Procédé selon la revendication 1, dans lequel le réseau neuronal de prétraitement comprend des couches convolutives récurrentes successives.

3. Procédé selon la revendication 2, dans lequel les couches convolutives récurrentes sont mises en oeuvre sous forme de bancs de filtres récursifs.

4. Procédé selon la revendication 1, dans lequel le réseau neuronal de prétraitement comprend un bloc de traitement d'enveloppe configuré pour calculer des enveloppes de signal de sous-bande pour le signal d'entrée audio.

5. Procédé selon la revendication 1, dans lequel le réseau neuronal de prétraitement comprend une couche de regroupement configurée pour la décimation de signal dans le réseau neuronal de prétraitement.

6. Procédé selon la revendication 1, dans lequel la classification initiale du signal est un vecteur de caractéristiques multidimensionnel.

7. Procédé selon la revendication 1, dans lequel le réseau neuronal classificateur de scène comprend une couche de réseau neuronal entièrement connectée.

8. Système selon la revendication 1, dans lequel le réseau neuronal classificateur de scène comprend un classificateur d'analyse discriminante linéaire (LDA).

9. Procédé de traitement de signal pour générer des signaux de stimulation pour un implant auditif (305) implanté chez un patient, le système comprenant :

un classificateur de scène audio (601) comprenant un réseau neuronal multicouche configuré pour classer un signal d'entrée audio à partir d'une scène audio, dans lequel le classificateur de scène audio comprend :

a) un réseau neuronal de prétraitement (603) configuré pour prétraiter le signal d'entrée audio sur la base de paramètres de classification initiaux pour produire une classification initiale du signal, et
b) un réseau neuronal classificateur de scène (604) configuré pour traiter la classification initiale du signal sur la base des paramètres de classification de scène pour produire une sortie de classification de scène audio,

dans lequel les paramètres de classification initiaux reflètent l'entraînement du réseau neuronal sur la base d'un premier ensemble de données d'entraînement audio initiales, et les paramètres de classification de scène reflètent l'entraînement du réseau neuronal sur un deuxième ensemble de données d'entraînement audio de classification distinctes et différentes du premier ensemble de données d'entraînement audio initiales, dans lequel le réseau neuronal de prétraitement (603) optimise les méta-paramètres sans entraînement explicite des poids par rétropropagation, et dans lequel le réseau neuronal classificateur (604) comprend une procédure de rétropropagation ;
un processeur de signal d'implant auditif (602) configuré pour traiter le signal d'entrée audio et la sortie de classification de scène audio pour générer les signaux de stimulation.

10. Système selon la revendication 9, dans lequel le réseau neuronal de prétraitement comprend des couches convolutives récurrentes successives.

11. Système selon la revendication 10, dans lequel les couches convolutives récurrentes sont mises en oeuvre sous forme de bancs de filtres récursifs.

12. Système selon la revendication 9, dans lequel le réseau neuronal de prétraitement comprend un bloc de traitement d'enveloppe configuré pour calculer des enveloppes de signal de sous-bande pour le signal d'entrée audio.

13. Système selon la revendication 9, dans lequel le réseau neuronal de prétraitement comprend une couche de regroupement configurée pour la décimation de signal dans le réseau neuronal de prétraitement.

**14.** Système selon la revendication 9, dans lequel la classification initiale du signal est un vecteur de caractéristiques multidimensionnel.

**15.** Système selon la revendication 9, dans lequel le réseau neuronal classificateur de scène comprend une couche de réseau neuronal entièrement connectée ou
dans lequel le réseau neuronal classificateur de scène comprend un classificateur d'analyse discriminante linéaire (LDA).

*Fig. 1*

**Fig. 2**

Fig. 3

**Fig. 4**

**Fig. 5**

*Fig. 6*

INITIALIZE PRE-PROCESSING NN WITH PRE-CALCULATED PARAMETER ESTIMATES — 701

SELECT A TRAINING SET — 702

INPUT TRAINING SET TO PRE-PROCESSING NN — 703

USE OUTPUTOF PRE-PROCESSING NN FOR OPTIMIZING CLASSIFIER NN — 707

*Fig. 7*

USE OPTIMIZED PARAMETERS FROM STEP 1 FOR CLASSIFIER NN — 801

SELECT A TRAINING SET, DIFFERENT FROM TRAINING SET IN STEP 1 — 802

INPUT TRAINING SET TO PRE-PROCESSING NN — 803

USE OUTPUT OF PRE-PROCESSING NN FOR OPTIMIZING CLASSIFIER NN — 804

DETERMINE ERROR VECTOR FROM CLASSIFIER NN OUTPUT — 805

USE ERROR VECTOR FOR OPTIMIZING PRE-PROCESSING NN PARAMETERS — 806

*Fig. 8*

**Fig. 9**

**Fig. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6348070 B
- US 7561709 B
- WO 2010085477 A
- CN 108231067 A

### Non-patent literature cited in the description

- **VERMEIRE et al.** Neural tonotopy in cochlear implants: An evaluation in unilateral cochlear implant patients with unilateral deafness and tinnitus. *Hear Res,* 12 September 2008, vol. 245 (1-2), 98-106
- **SCHATZER et al.** Electric-acoustic pitch comparisons in single-sided-deaf cochlear implant users: Frequency-place functions and rate pitch. *Hear Res,* March 2014, vol. 309, 26-35
- **HOCHMAIR I ; NOPP P ; JOLLY C ; SCHMIDT M ; SCHÖßER H ; GARNHAM C ; ANDERSON I.** MED-EL Cochlear Implants: State of the Art and a Glimpse into the Future. *Trends in Amplification,* 2006, vol. 10, 201-219
- **LORENS et al.** Fine structure processing improves speech perception as well as objective and subjective benefits in pediatric MED-EL COMBI 40+ users. *International journal of pediatric otorhinolaryngology,* 2010, vol. 74.12, 1372-1378
- **VERMEIRE et al.** Better speech recognition in noise with the fine structure processing coding strategy. *ORL,* 2010, vol. 72.6, 305-311
- **GERHARD, DAVID.** Pitch extraction and fundamental frequency: History and current techniques. Regina: Department of Computer Science, University of Regina, 2003
- **FONTAINE et al.** Brian Hears: Online Auditory Processing Using Vectorization Over Channels. *Frontiers in Neuroinformatics,* 2011
- **DELIANG WANG.** Deep Learning Reinvents the Hearing Aid. *IEEE Spectrum,* 01 December 2016, 1-8
- The CMA evolution strategy: A comparing review. **N. HANSEN.** Towards a new evolutionary computation. Advances in estimation of distribution algorithms. Springer, 2006, 75-102
- **H. BEYER.** Theory of Evolution Strategies. Springer, 2001
- **S. COLUTTO ; F. FRÜHAUF ; M. FUCHS ; O. SCHERZER.** The CMA-ES on Riemannian manifolds to reconstruct shapes in 3-D voxel images. *IEEE Transactions on Evolutionary Computation,* April 2010, vol. 14 (2), 227-245
- **ALEXANDER FORRESTER ; ANDRAS SOBESTER ; ANDY KEANE.** Engineering Design via Surrogate Modeling: A Practical Guide. Wiley, September 2008
- **CLAUS WEIHS ; SWETLANA HERBRANDT ; NADJA BAUER ; KLAUS FRIEDRICHS ; DANIEL HORN.** Efficient Global Optimization: Motivation, Variations, and Applications. *ARCHIVES OF DATA SCIENCE,* 2016